# EUROPEAN PATENT APPLICATION

(11) **EP 1 494 158 A2**
(43) Date of publication of application: **05.01.2005**
(21) Application number: 04015067.4
(22) Date of filing: 26.06.2004
(51) Int. Cl.: G06F 19/00

(54) **Method and apparatus for managing data received from a medical device**

(30) Priority: 30.06.2003 US 483246 P; 30.06.2003 US 483248 P; 30.06.2003 US 483249 P; 30.06.2003 US 483250 P; 30.06.2003 US 483251 P; 30.06.2003 US 483252 P; 30.06.2003 US 483253 P
(71) Applicant: Becton Dickinson and Company, Franklin Lakes, NJ 07414-1880 (US)
(72) Inventor: Arndt, Dave, North Caldwell NJ 07006 (US); Upham, Paul, Edgewater, NJ 07020-1289 (US); Gibney, Michael, Chestnut Ridge NY 10977-6602 (US); Gravel, Marian, Newburgh NY 12550 (US); Friedel, Betty, Bergenfield NJ 07621 (US); Yao, Raymond, Hoboken NJ 07030-4957 (US); Puma, Michael, Midland Park NJ 07432 (US); Grauer, Carl, Brooklyn NY 11215 (US); Griffin, Phyllis, Ringwood NJ 07456 (US)
(74) Representative: Selting, Günther, Dipl.-Ing.

(57) **Abstract**

Embodiments of the present invention provide the user enhanced capabilities in managing data measured by a medical device. The user is able to create customized graphic displays of measured data, update and store the graphic displays. Additionally, embodiments of the present invention provides the capabilities to share the data with healthcare professionals and includes functionality that allows multiple reports to be printed for multiple patients. In addition, interactive displays allow data in one display format to be displayed in another. Finally, embodiments of the present invention has the capability to provide tutorials for important features of the present invention

## Description

This application claims the benefit of U.S. Provisional Application No. 60/483,246, U.S. Provisional Application No. 60/483,248, U.S. Provisional Application No. 60/483,249, U.S. Provisional Application No. 60/483,250, 60/483,251, U.S. Provisional Application No. 60/483,252, and U.S. Provisional Application No. 60/483,253, all of which were filed on June 30, 2003, the entire content of each of said applications being incorporated herein by reference. Related subject matter is disclosed and claimed in a concurrently-filed U.S. nonprovisional patent application of Scott Gisler et al. entitled "Self Powered Serial-to-Serial or USB-to-Serial Cable with Loopback and Isolation", Attorney Docket No. 47057 (P-5762), the entire content of which is incorporated herein by reference

### Field of the Invention

The present invention relates to the management and display of medical data. More particularly, the present invention relates to a base device for receiving data from a medical device, managing the data and displaying the data, which allows a user to manage the medical condition monitored by the medical device. The preferred medical device for the present invention is a blood glucose monitor.

### Background of the Invention

Medical condition monitoring devices, such as blood glucose monitors, collect and store a large amount of data. The data stored and collected by these devices may be used to analyze the data of the patients whose data was collected for a number of reasons. Analysis can provide trend information as the patients condition changes, the patient's reaction to different drugs or drug dosages, and raw data that must be placed in an algorithm for further analysis. The data may also be displayed to the patient or the patient's healthcare provider for interpretation. Graphical interpretation allows the viewer to better analyze trends and other more complex data.

The graphical information may be presented to users in a variety of formats. In displaying data related to diabetes, for instance, the graphical format is usually presented in a standard day format. The standard day format, also called the modal day and the average day, presents the diabetic's blood glucose measurements for the day. In addition, the display devices may also present the dosages and type of insulin administered during the day. The graphical data may also be presented in numerical chart formats, bar graph formats, pie chart formats, line charts, and the like.

Typically, however, the user may only use the graph formats selected by the medical device vendor or by a third-party software vendor. Since each provider presents the data in a different manner this makes it difficult for the healthcare provider to interpret each patient's data because each patient may be using a different vendor's device or software or even different devices with software different from the device vendor's. Additionally, neither the healthcare professional nor the patient may customize the graphical display in a manner of their choosing.

Presently, there are many systems that allow a user to download information to a base device and view the data graphically. However, the functionality of those systems is lacking. The systems lack configurability by the user to place the data in a graphical format that is better understood by that particular user. Present systems lack the capability to allow the user to change settings and present data in multiple graphical formats in a single display. Furthermore, the present systems do not allow users, such as healthcare professionals or patients, to produce multiple reports with the single press of a button or the capability to easily reassign medical devices between patients.

Once the healthcare provider has a standard graphical display customized format for all of its patients, the prior art requires individual printing of each report for each patient. The prior art does not have a large scale batch printing feature available, which results in longer preparation times for the healthcare provider as well as more opportunities for errors to be made. Also, patients cannot with minimal interaction with the software application print out the graphical displays of multiple charts. The prior art also does not provide the user the ability to quickly print reports pertaining to downloaded new data.

The prior art also makes it very difficult to reassign monitors from a patient to another patient. Typically, the healthcare professional or patient must enter patient personal data and assign a name for or serial number of the medical device to the patient. If for whatever reason this data is entered incorrectly when data is downloaded from the medical device, the data may not be added to previously entered data for the patient. This occurs because the database file only accepts data for a specific patient from the initially assigned medical device. The software application will not allow commingling of data from other meters. This can be problematic when a patient uses different medical devices to track the medical condition or if the user switches medical devices for another reason. In those scenarios, the new data must be analyzed without the benefit of the historic data from the previous medical device.

In the case of blood glucose monitoring, some prior art software applications offer the display of blood sugar and insulin data in standard day chart format, but they do not offer a combination of two standard day charts in a single display. The prior art does not offer the ability to toggle between the display of blood sugar averages, ranges, standard deviations and individual blood sugar values, and with dynamically generated insulin labels in the legend.

The prior art also lacks the capability to easily show a different chart format while viewing another chart format. The prior art does not provide the capability to quickly with minimal interaction between the user and software application to convert information in one graphical display format into another graphical display format. For instance, the prior art does not provide the capability to view a standard day display and provide a pie chart display overlaid on the standard day display.

Finally, the prior art does not provide interactive tutorials that allow the user of the software application to view a step in the tutorial and then perform the same step in the software application. The prior art provides users with minimal tutorials with respect to software applications. Existing medical device download software does not provide interactive tutorials related to the use of the software or how to connect the device. The importance and value of software functions related to the blood glucose monitor merits an interactive tutorial devoted to creating custom graphical display layouts.

It would be advantageous if the healthcare provider could format a graphical data representation for all its patients in a single customizable format. Additionally, it would be convenient if the software application would provide a batch printing feature that would allow the healthcare professional the option to print out multiple reports for multiple patients with a single press of a button or click of a mouse. A further convenience would be found in the patient's being able to configure their own displays so that they too could better understand the data provided and the effects of treatment on their medical condition.

### Summary of the Invention

Embodiments of the present invention allow the user to create customized displays called Self Care Pages (SCP) that allow the user to customize their graphical displays in a layout that is the most informative to them. Additionally, the patient may customize the SCP with graphs that they are most comfortable reading, understanding and using in order to better explain how their treatment is progressing.

The graphical display formats present data in easy to understand formats. In the case of blood glucose monitoring, the standard day blood glucose and standard day insulin displays of embodiments of the present invention directly correspond. It is an advantage for the healthcare professional or patient (hereinafter the user) to be able to view both data sets in the same display. It is also an advantage that additional information regarding data for blood glucose and insulin measurements is presented in a graphical format that provides the most information without being overwhelming or overly busy looking.

The standard day chart, either for blood glucose or insulin, is the most difficult chart for patients to interpret. Conversely, pie chart graphic display formats seem to be more easily interpreted by patients. The present invention allows the user to select a timeframe from within the standard day chart and have the data for that timeframe displayed as a pie chart. This aids the patient in interpreting the data by providing the data in an alternative format. Additionally, the patient can easily select another timeframe for display as a pie chart from the standard day chart to compare the data.

The ability of users to easily change the assignment of medical devices from one user or add additional medical devices to a single user is an advantage of embodiments of the present invention. This function allows for commingling data from multiple meters in a single user database file thereby allowing the graphical displays to integrate medical condition measurement data from several medical devices for a better historical perspective of the user's medical condition.

The batch printing capability of embodiments of the present invention allows both types of users, patient and healthcare professional, to quickly print out multiple reports with a single click of a button. This saves time and allows the user to always have the same reports for analyzing a specific period of time. The healthcare professional has the ability to print out multiple patients' reports and the patient has the capability to print out several or all of the reports available to them. This allows the patient to print out a report for a specific time period, preferably the most recent two weeks, to provide to their healthcare professional for analysis.

Finally, the interactive tutorials of embodiments of the present invention teach users how to create their own Self Care Pages as well as how to connect a medical device to a base device using a data transfer cable with a 9-pin serial or Universal Serial Bus (USB) connector or both.

The above advantages and others will be made more evident in light of the following description in conjunction with the appended drawings.

### Brief Description of the Drawings

The invention will be more readily understood with reference to the embodiments thereof illustrated in the attached drawing figures, in which:

Fig. 1 is an exemplary configuration of the system for which data will be managed by an embodiment of the present invention;

Fig. 2 shows an exemplary Self Care Page created by an embodiment of the present invention;

Fig. 3 shows an exemplary display resulting from the Create New operation when creating a Self Care Page in accordance with an embodiment of the present invention;

Fig. 4 shows an exemplary display resulting from the Enter the SCP Name operation when creating a Self Care Page in accordance with an embodiment of the present invention;

Fig. 5 is a flowchart of how a user would create the exemplary Self Care Page of Fig. 1 according to an embodiment of the present invention;

Fig. 6 is an exemplary view of a default Self Care Page with a drop down menu for selecting other Self Care Pages according to an embodiment of the present invention;

Fig. 7 is a flowchart of the steps for a user to view a Self Care Page according to an embodiment of the present invention;

Fig. 8 shows an exemplary default view of the Blood Sugar Standard Day and the Insulin Standard Day in a single display according to an embodiment of the present invention;

Fig. 9 shows an exemplary blood sugar data points view of the Blood Sugar Standard Day and the Insulin Standard Day in a single display according to an embodiment of the present invention;

Fig. 10 shows an exemplary view of the Blood Sugar Standard Day and the Insulin Standard Day with an exemplary dynamic legend window in a single display according to an embodiment of the present invention;

Fig. 11 is a flowchart of the steps for a user to view a Blood Sugar and Insulin chart according to an embodiment of the present invention;

Fig. 12 shows an exemplary view of the Standard Day-Blood Sugar display from which a pie chart window may be displayed according to an embodiment of the present invention;

Fig. 13 is an exemplary image of the pie chart window according to an embodiment of the present invention;

Fig. 14 is a flowchart of the steps in creating a pie chart from Standard Day-Blood Sugar display according to an embodiment of the present invention;

Fig. 15A is an exemplary image of the meter manager function for breaking an assignment of a medical device to a patient according to an embodiment of the present invention;

Fig. 15B is an exemplary image of the meter manager message shown to users verifying the unassignment request and indicating the results when a meter will be unassigned according to an embodiment of the present invention;

Fig. 16 is a flowchart of the steps in removing an assignment of the medical device from a medical device user according to an embodiment of the present invention;

Fig. 17 is an exemplary image of the Home Page the one-click download and recording function according to an embodiment of the present invention;

Fig. 18 is a flowchart of the steps for downloading and printing data from a medical device according to an embodiment of the present invention;

Fig. 19A is an exemplary image of the batch printout window according to an embodiment of the present invention;

Fig. 19B is an exemplary image of the batch printout window when a user selects a particular function according to an embodiment of the present invention;

Fig. 20 is a flowchart of the steps for navigating the printout window for printing reports according to an embodiment of the present invention;

Fig. 21 is a flowchart for navigating through the selection to the interactive tutorials according to an embodiment of the present invention.

In the drawing figures, it will be understood that like numerals refer to like features and structures.

### Detailed Description of the Preferred Embodiments

Various embodiments of the invention will now be described with reference to a blood glucose meter as the preferred medical device.

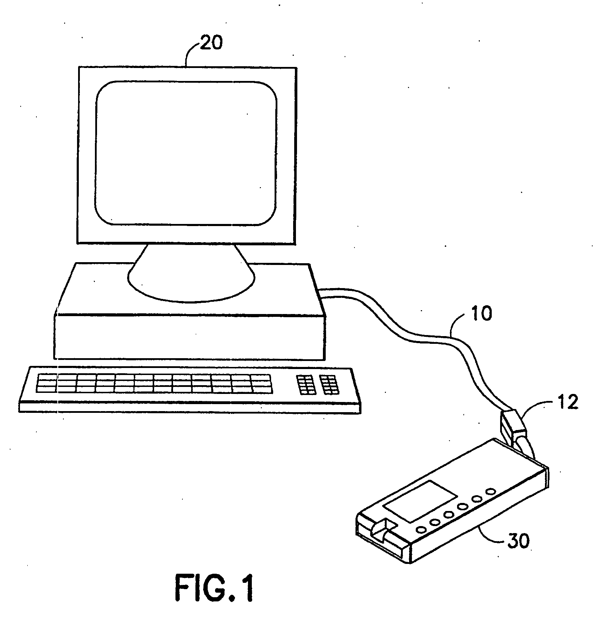

Fig. 1 is an exemplary configuration of a system that implements an embodiment of the present invention. The system comprises a base device 20, in this case a personal computer (PC), a data transfer cable 10 with electronics 12, and a medical device 30, such as a blood glucose monitor. For discussion purposes, the preferred medical device 30 will be a blood glucose meter and the data discussed will generally relate to the treatment of diabetes. However, the invention should not be limited to receiving data from only a blood glucose meter and for the treatment of diabetes.

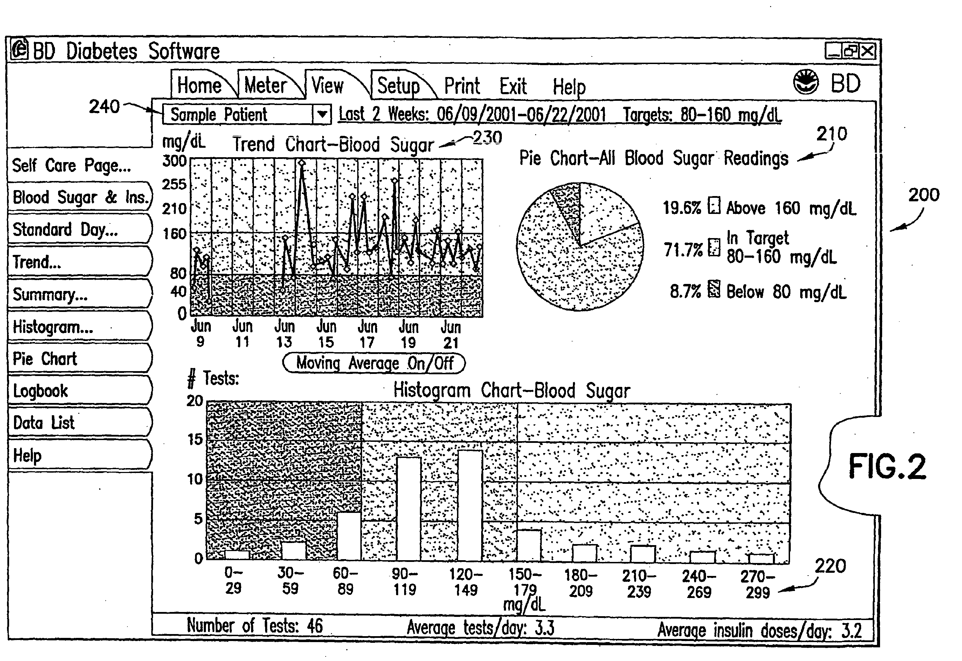

The PC 20 downloads blood glucose measurement data from the blood glucose meter 30 for analysis and display to healthcare professionals and patients. A patient is a person whose blood glucose was measured and stored in the blood glucose meter 30. The patients and healthcare professionals need to see and analyze the measured data to verify the effects of an insulin treatment regime. All users, both healthcare professionals and patients alike more easily interpret graphical representations, than rigid numerical tables. Referring to Fig. 2, graphical representations (210, 220, and 230) also make it easier for patterns or trends to be easily identified. To facilitate creating easy to understand graphical representations, an embodiment of the present invention provides the user the ability to configure the data in views easily understood by the user. Preferably, the embodiment of the invention is implemented in a familiar web browser interface that guides the user through the creation of these views. In the default Self Care Page (SCP) 200 of Fig. 2, the patient's name is Sample, Patient (240). Sample's SCP 200 has a pie chart 210 of blood sugar readings, a histogram chart 220 of actual blood measurements in mg/dl, and a trend chart 230 of blood sugar readings for the last two weeks.

The SCP 200 is a user-configurable view of data that provides a means to display up to three different graphs on one screen from a choice of nine graphs. The user can create as many SCP 200 as they want. The creation of each Self Care Page 200 is performed using a step-by-step configuration method, prompting the user for input at each step in the process and providing graphical and text feedback to confirm their choices.

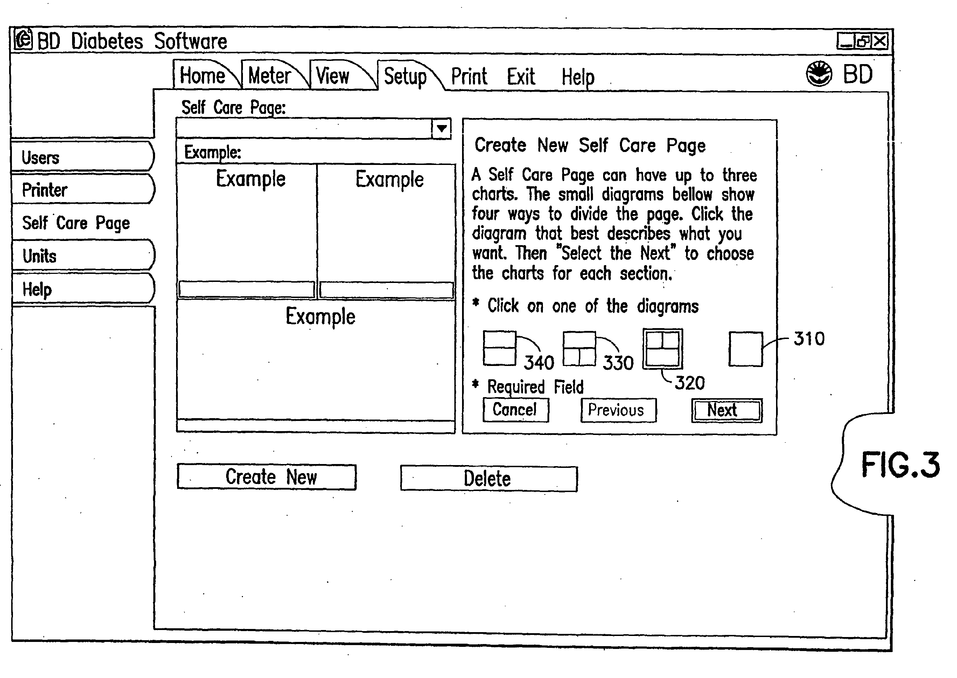

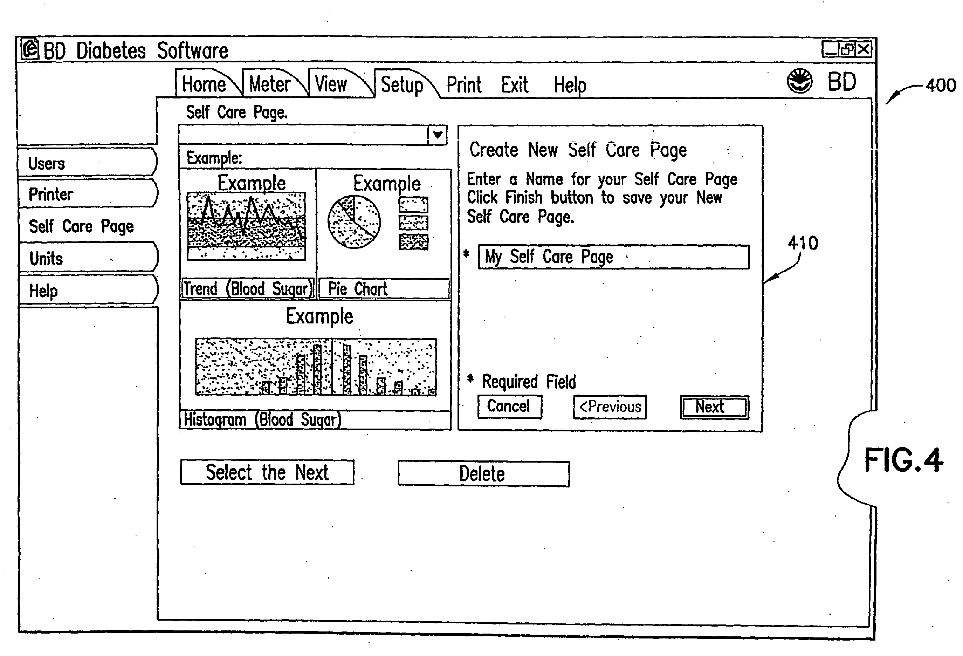

Referring to Fig. 3, when customizing the SCP 200, the user preferably has a number of choices for the configuration such as, preferably: (1) only one graph 310, (2) three graphs with two on the top and one on the bottom 320, (3) three graphs with one on the top and two on the bottom 330, and (4) two graphs with one on the top and one on the bottom 340. For any configuration, the user is prompted to choose from the nine available graphs for each section of the display. Once the user has chosen all of the graphs desired, as shown in Fig. 4, the user provides a name in the required field box 410 of the display 400 for the SCP 200 just created.

The default data view for the software is one of three SCP 200 that are automatically configured when the software installs as shown previously in Fig. 2. The three default SCP's cannot be modified or deleted. When the user creates additional SCP's, the SCP's are listed in alphanumeric order following the default pages in the SCP's selection list. When the user clicks on a SCP 200 from the list, the software displays the currently selected patient's data in the selected configuration. Each individual graph that displays in the SCP 200 retains all of its unique interactive functionality.

When a SCP is printed, the selected graph configuration is printed on a single page.

The programming routines used to create SCP 200 will now be described in more detail. A BDLayout.Report control provides the user interface display widget for displaying sample self care page layouts to the user. The BDLayout.Report control hosts three instances of a BDLayout.Selection control.

A BDSetup.Report control provides the user interface host container for the self care page setup feature. This control implements a BDObject interface. The implementation of this interface controls the interaction of the control with the navigation framework.

The report control interfaces with a business object layer (BO) using a BDViewUtil.XmlTrans object.

The report component uses a XMLTrans component ("BDViewUtil.XmlTrans") to fetch/return Report information from the BDCustomReport business object.

When displaying a SCP, a BDView.Container receives a message from a BD.Navigator component that it should display a SCP, it activates each of the chart components defined for that SCP. The charts, in turn, draw their chart displays in the areas of the screen that have been assigned to them.

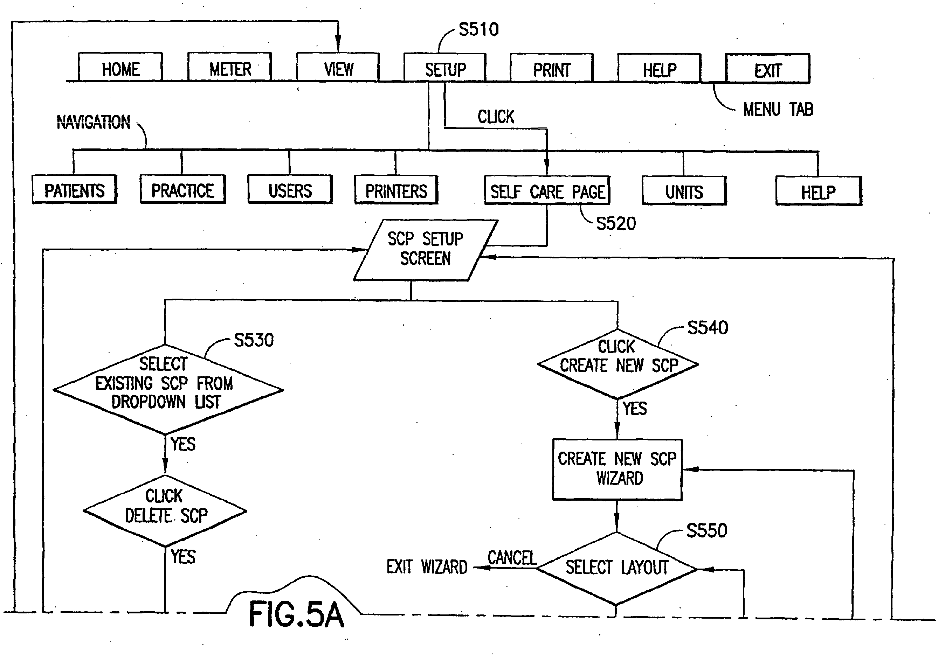

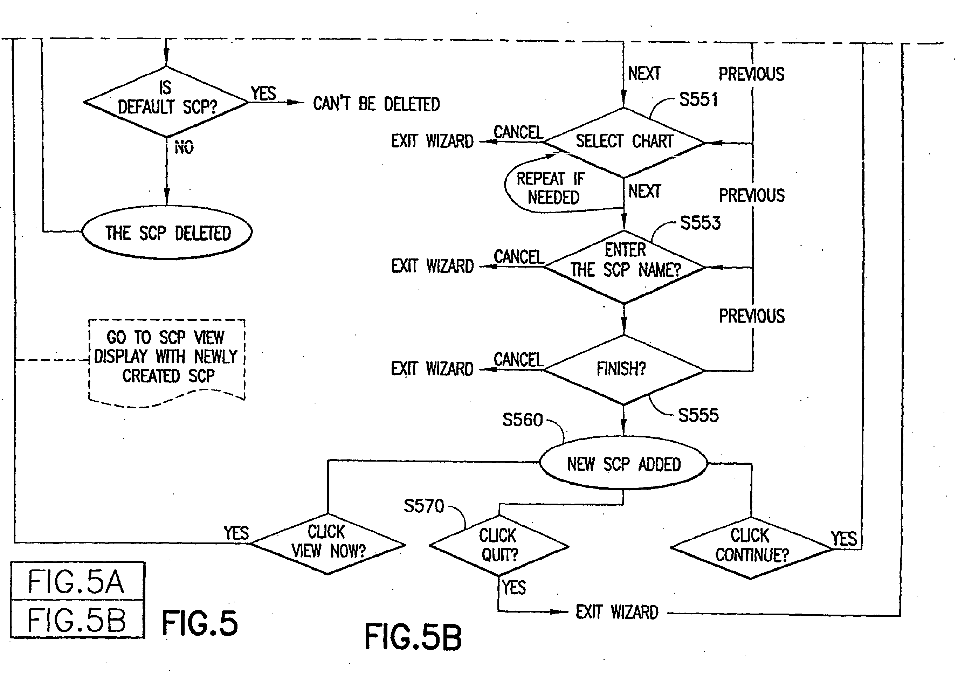

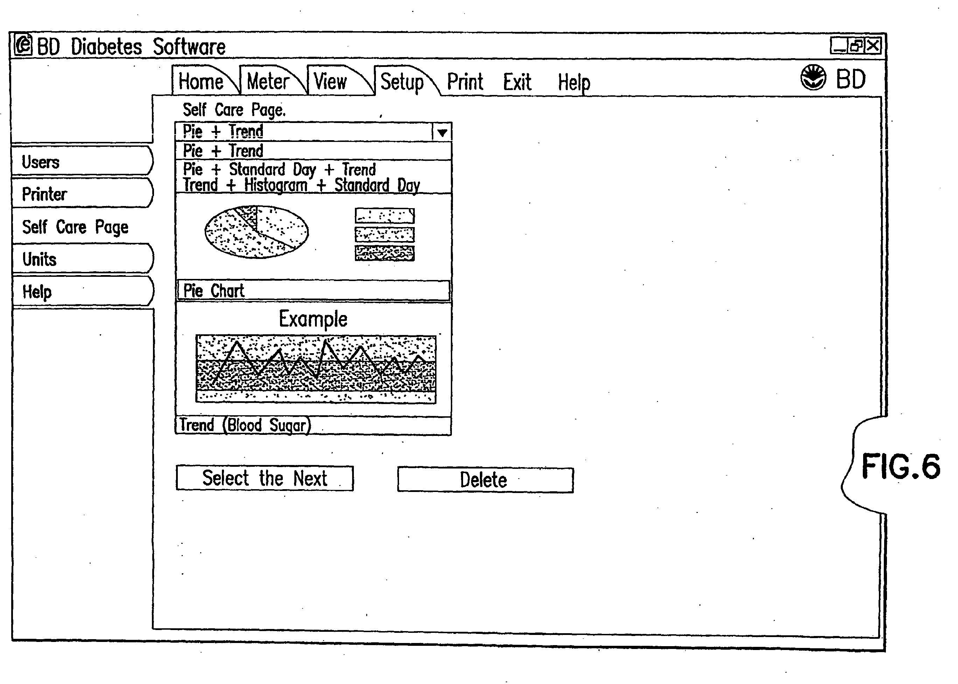

Fig. 5 shows the user path for creating a new SCP 200. Beginning at the Setup tab (S510), the user clicks on SCP (S520) and then has a choice between selecting an existing non-default SCP (S530) or creating a new SCP (S540). The image of this display is shown in Fig. 6. The user is given the option to select the configuration or layout of the graphs (S550). After selecting the layout of the graphs, the user must select which of the nine available graphs will be shown in the selected layout (S551). After completing the configuring and selecting of the graphs, the user must name the SCP (S553). The user will then be asked if he or she has finished (S560). Once the user has created a new SCP 200, the user can continue to make additional SCPs, quit and stay on the Setup screen, or view the SCP 200 just created (S570).

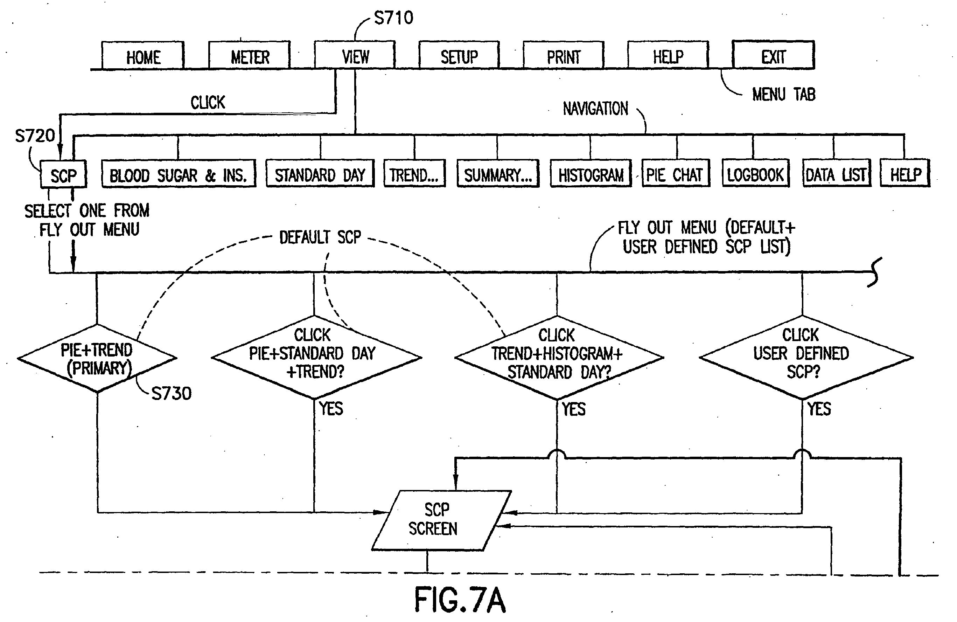

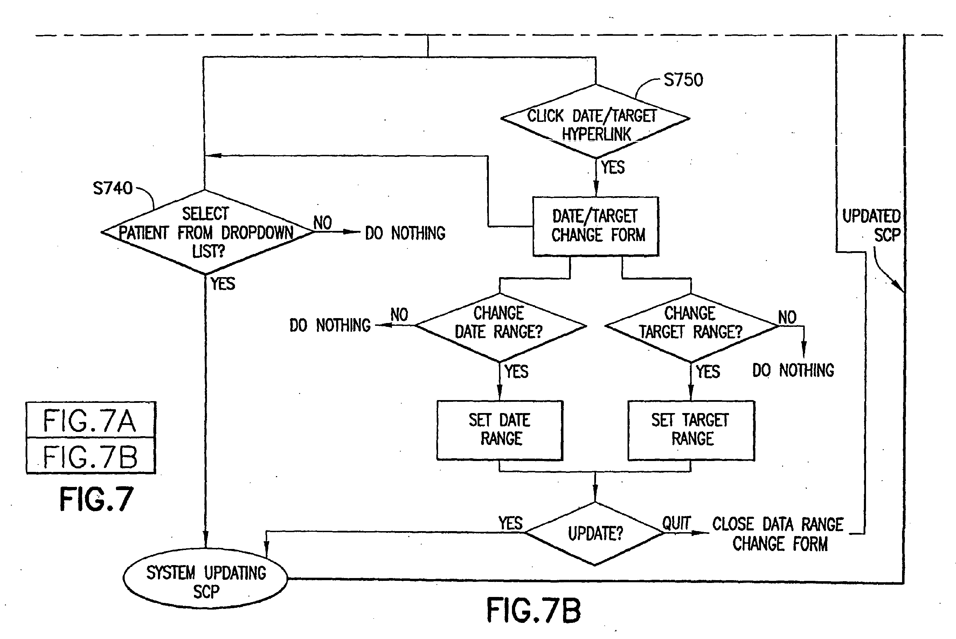

Fig. 7 shows the user steps for displaying any of the available SCPs. From the Home Page, the user selects the View tab (S710), the user is then presented with a number of choices. To view a SCP, the user selects the SCP menu choice (S720). The user then selects from a menu of default SCP or previously user defined SCP from a fly out menu (S730). The ability to select patients (S740) and change date ranges and targets (S750) is available from all Views.

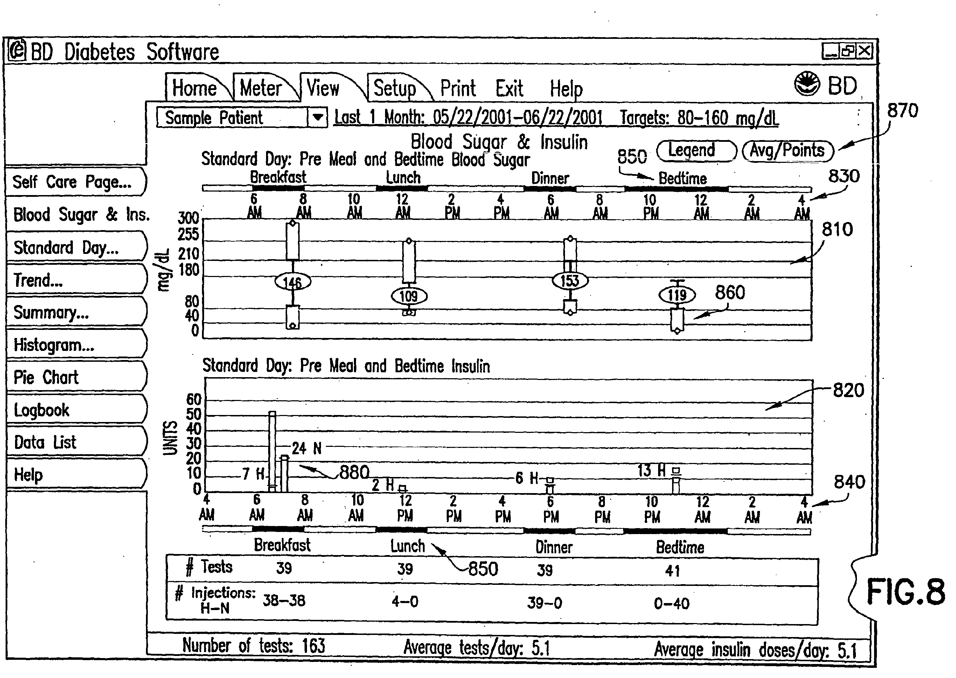

A common view used by most users is the data display that shows both blood sugar and insulin standard day data in a single display. Referring to Fig. 8, the blood sugar 810 is displayed in a standard day format where the selected blood sugar data are plotted on a 24-hour clock 830. Beneath the blood sugar standard day is the insulin dose standard day graph 820. The insulin data are preferably displayed using the same 24-hour clock 840 so that the average insulin doses 880 for each type of insulin are visually represented directly below the blood sugars from the same time period. Additionally, the custom meal times 850 are displayed graphically as colored, horizontal bars both above the blood sugar portion and below the insulin portion.

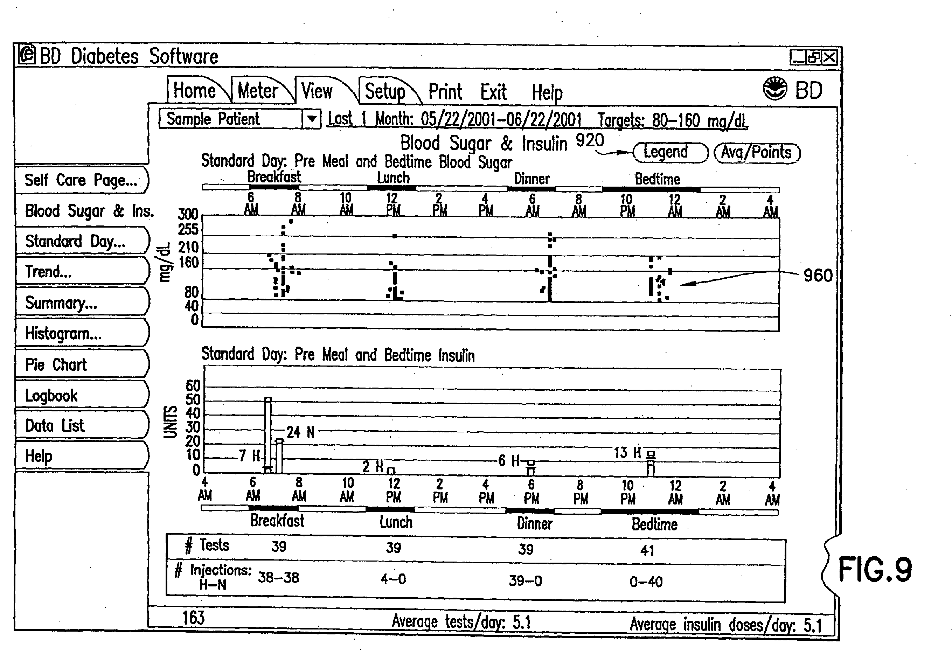

In the blood sugar portion of the display 810, the user can switch between two views of the blood sugar data. In the default view, the blood sugar data are represented as averages by mealtime and bedtime 860. In addition, the range of values represented by the small circles above and below the average blood sugar value shown in the ovals and the standard deviation of the average is represented graphically by a stretched I-like indicator bar for each mealtime and bedtime. The second view is triggered by the user clicking on the Averages/Points button 870. As shown in Fig. 9, this changes the appearance of the blood sugar graph so that it shows the individual blood sugar values 960 rather than the averages, ranges, and standard deviations.

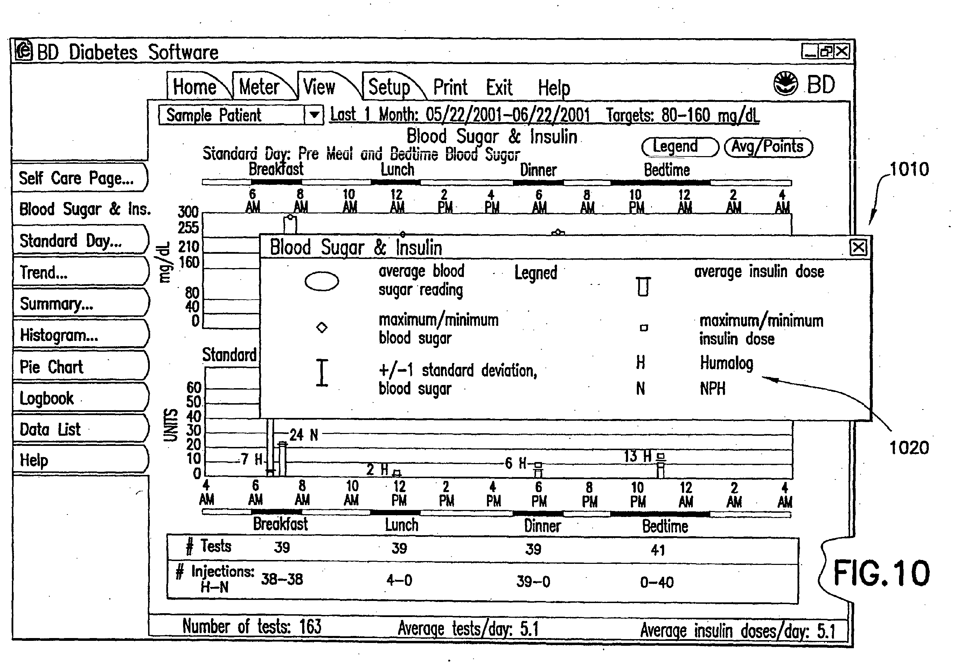

The user can also click on a Legend button 920 to display a legend that describes the graph elements for both the blood sugar and insulin displays. Referring to Fig. 10, in the legend 1010, the description of the insulin 1020 doses is dynamically generated based on the insulin data that the user has provided. That is, if the user has one or more insulin types 1020 recorded, the legend 1010 will automatically display only those insulin 1020 labels that are linked to that user.

Referring back to Fig. 8, this data display simplifies the depiction of two critical pieces of information for pattern detection in diabetes management. Rather than showing all the individual data points for the blood sugar 810 and insulin 820 data, embodiments of the present invention show averages by mealtimes and bedtime. Variation is represented graphically rather than numerically aiding the detection of patterns in the data. Also, the juxtaposition of these unique displays 810, 820 facilitates the user's ability to make a mental association between the blood sugar values 860 and the insulin doses 880.

In a preferred embodiment of the present invention, the component of software that implements the blood glucose and insulin chart is preferably an Active X Full Control COM DLL named BDModalDay.dll. Its ProgId may be "BDModalDay.Chart". The container will instantiate this chart when the user requests it via a menu choice, and will tell it which patient, which date range, and which data category (BD_GLUCOSE|BD_INSULIN) is requested. The chart then will obtain the appropriate data. It will create a transaction object, and that object in turn will fetch the data points, and will preferably return them as Extensible Markup Language (XML). The choice of using XML is a design choice. Those of skill in the art will appreciate that any suitable formats, including other markup languages such as SGML, may also be used. The data will be further organized and stored in member variables.

When the software receives a message to create a display as shown in Fig. 8, it will preferably consult the data it has in member variables and will use it to populate the charts shown in the displays.

The chart will expose the interfaces IDispatch, IChart, and IBDObject to its container.

The primary component of the Blood Glucose and Insulin chart will preferably be an Active X Full Control COM DLL named BDModalDay.dll, preferably implemented in the C++ programming language. Its ProgId can be "BD.ModalDay.Chart".

The IChart interface is preferably used to instantiate the Standard Day Chart Control and to set its properties. The properties are used by the Standard Day Control to fetch the appropriate set of data points from the database, and format it especially for Standard Day viewing.

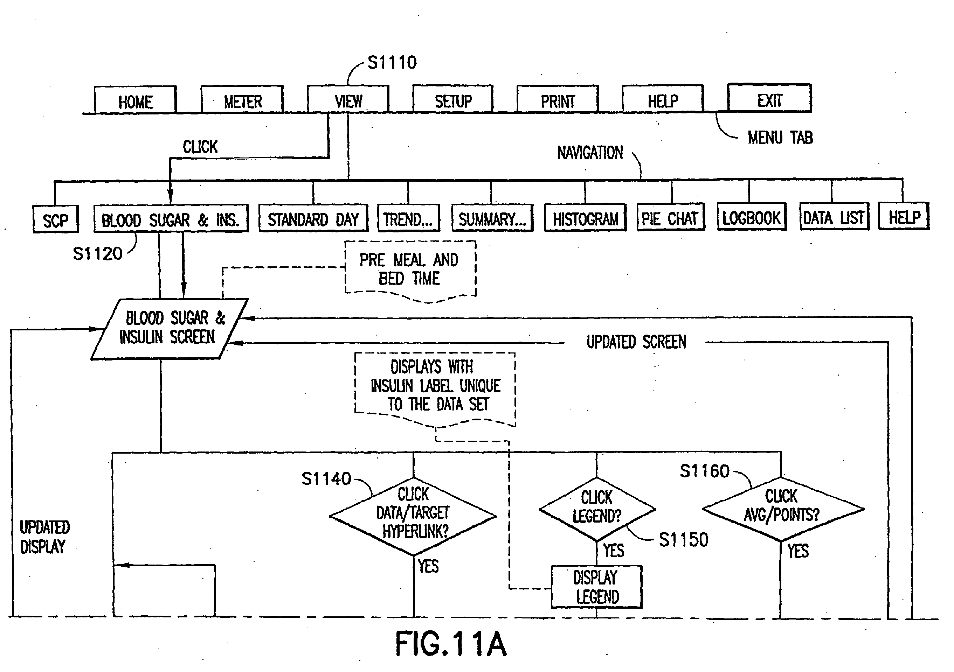

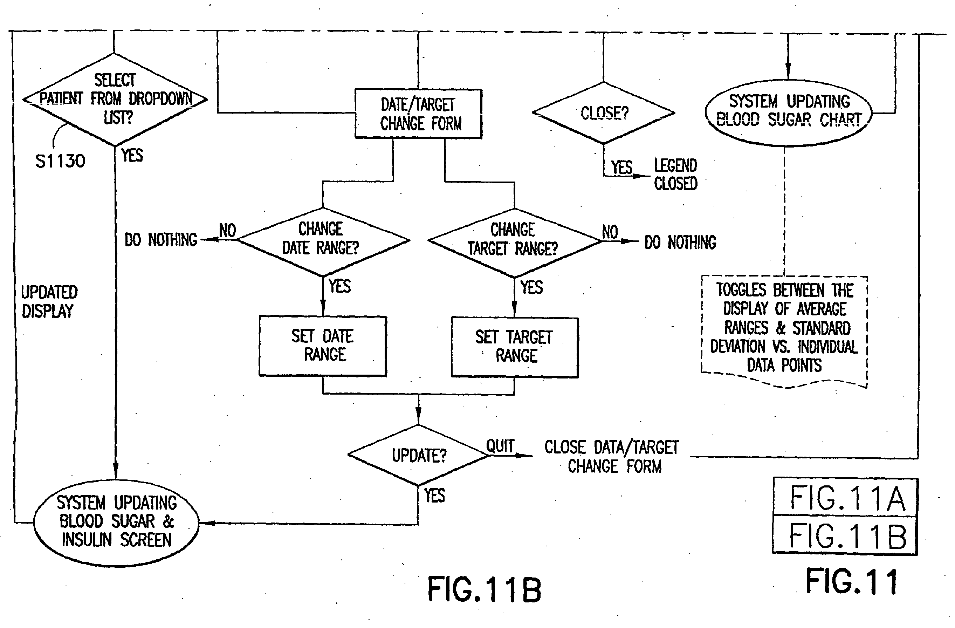

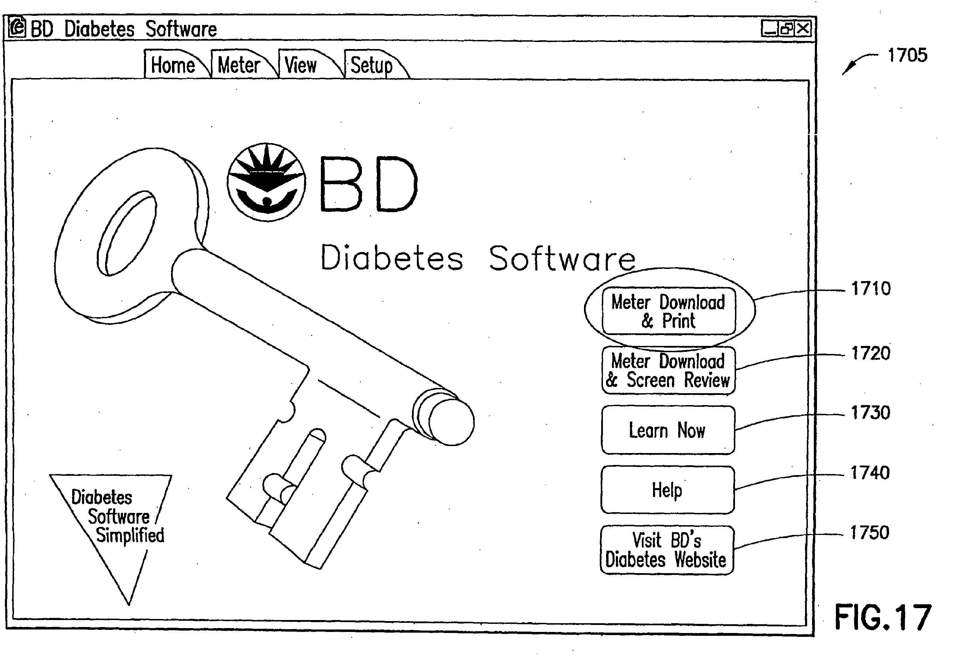

Fig. 11 shows exemplary steps by which a user accesses the "Blood Sugar & Insulin" chart in the software program. From the Home Page (see Fig. 17), the user selects the view menu tab (S 1110). This will present a navigation menu from which the user selects Blood Sugar and Insulin S1120 tab. From there, the user has the option to select a patient from a drop down menu S1130, change the date range or the target blood glucose range of the graph S 1140, the user could also choose to view the legend S1150 or change the blood sugar graph by choosing to view the average points S1160.

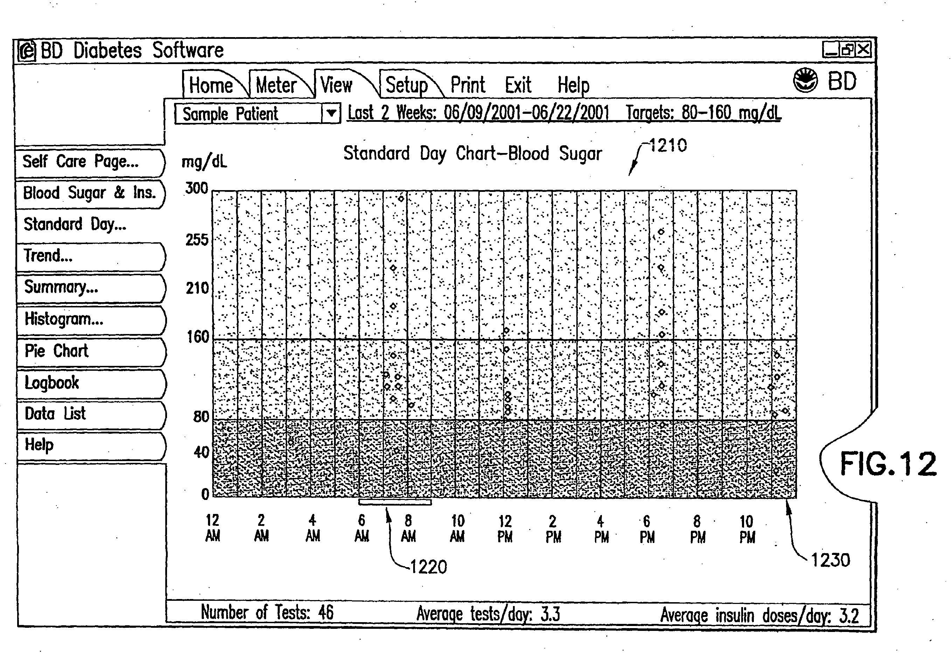

Embodiments of the present invention can include an alternative data display that is triggered from, for example, a standard day chart. Fig. 12 is an exemplary view of the standard day blood sugar chart. To trigger the alternative data display from the standard day blood sugar chart view 1210, the user places the cursor along the x-axis of this chart 1230. The cursor preferably changes to a pointing finger and a colored horizontal bar 1220, preferably blue, appears between the x-axis labels and the bottom of the data display. The x-axis 1230 preferably displays 24 hours of time. The horizontal bar's 1220 width preferably represents a 3-hour segment based on the width of the x-axis labels.

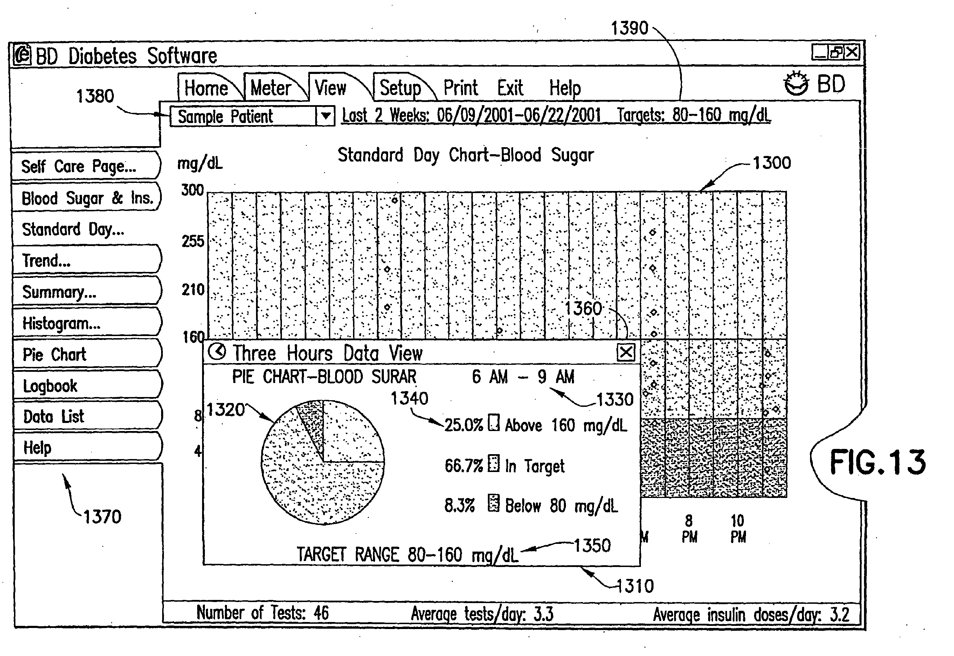

When the user clicks the mouse while the cursor is in this state and the blue bar is showing, the image shown in Fig. 13 is displayed, a small window 1310, the Three Hour Data View, appears with a pie chart 1320 whose data are based on the selected 3-hour period 1330. The pie chart 1320 shows the percent of blood sugar values 1340 that are above, within, and below the target range set for the displayed patient, within the selected 3-hour period 1330. The pie chart 1320 is labeled with the range of time represented 1330 and with the blood sugar target range 1350 for the selected patient. Underlying the Three Hour Data View 1310 is the Standard Day Blood Sugar Chart 1300.

Because the standard day chart 1300 is more difficult for patients to interpret than any of the other data displays, this invention provides a means for sampling a portion of the data that are displayed in the standard day chart 1300 to provide a more common view, such as the pie chart 1320, of this data sample.

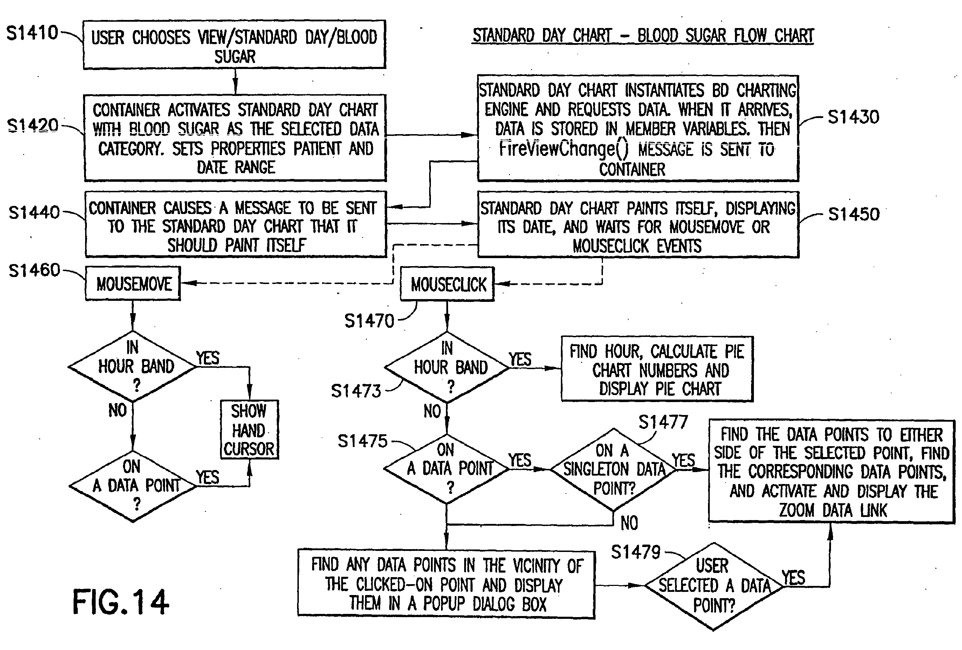

Fig. 14 outlines how the data for a Three Hour Data View window is determined. In step S 1410, the user chooses the view. In step S1420, a software routine activates the standard day chart with blood sugar as the selected data category. The software further sets the properties for the patient and date range. The software, at step S1430, instantiates the charting program and requests data. The data is supplied and stored in member variables. A message is then sent to the software routine that activated the standard day chart in step S 1420. Once the message of step S1430 is received, the software routine sends a message to the software routine controlling the display of the standard day chart. The message tells the software routine to start displaying the standard day chart 1300. In step 1450, the standard day chart 1300 is displayed and the software is waiting for an input from the user. The user can move the mouse as shown in step S 1460 over any three hour increments. The three-hour period of time is determined by the position of the user's mouse click in the bottom zone of the chart in step S 1470. The mouse click is interpreted as occurring in the middle hour. For each hour of the standard day, a total was kept of the number of readings, preferably unmarked data points only, below the target range, in the target range, and above the target range. Sums are formed of the total number of below range readings over the three-hour period, the total number of in range readings over the three-hour period, and the total number of above range readings over the three-hour period. Then a grand total of all readings over the three-hour period is formed. The percentage of below-target readings is calculated by dividing the number of below-target readings over the three-hour period by the grand total. Percentages of in-target and above-target readings are calculated similarly. This data is preferably then presented in the Three Hour Data View 1310 pie chart.

However, when there is a mouse click S 1470, it must be determined if the mouse click occurred in a grouping of data points in the Standard Day Chart-Blood Sugar S 1475. If so, then at step S 1477, it must be determined if the mouse click S 1470 occurred on a single data point. If the mouse click S 1470 is determined in step S 1477 to have occurred on a single data point, then the data points to either side of the selected point are found and displayed in a Zoom Data Link popup window (not shown). Also shown are the insulin values for all the data points shown. In step S 1477, if it is determined that mouse click S 1470 occurred on multiple data points, then the data points in the vicinity of the clicked-on point are displayed in a popup window (not shown) and the user is asked to choose a specific data point for which a Zoom Data Link popup window will be created.

Referring back to Fig. 13, clicking on the X in the upper right hand comer 1360 closes the Popup window 1310. If the user chooses a different type of graph from the menu 1370, or changes the patient 1380 or date range 1390, the popup window 1310 will disappear.

The software components for creating the Three Hour Data View 1310 pie chart will now be described in detail. The Three Hour Data View 1310 pie chart window will preferably be implemented as an ATL COM dialog box window that will be integrated into the Chart object of the BDPie component. It will be wrapped in a class of its own, CMDPiePopup. This class will use the template, CDialogImpl, so that it will have the default behavior of a dialog box. The Three Hour Data View window 1310 pie chart will be created as a modeless dialog box. It will have only the one active button, the X in the upper right hand comer that causes it to close. One instance of the CMDPiePopup class will be embedded in the Standard Day chart 1300.

The CMDPiePopup class is related to the containing Chart class in exactly the same way that the CzoomDataLink class is related to the containing Chart class.

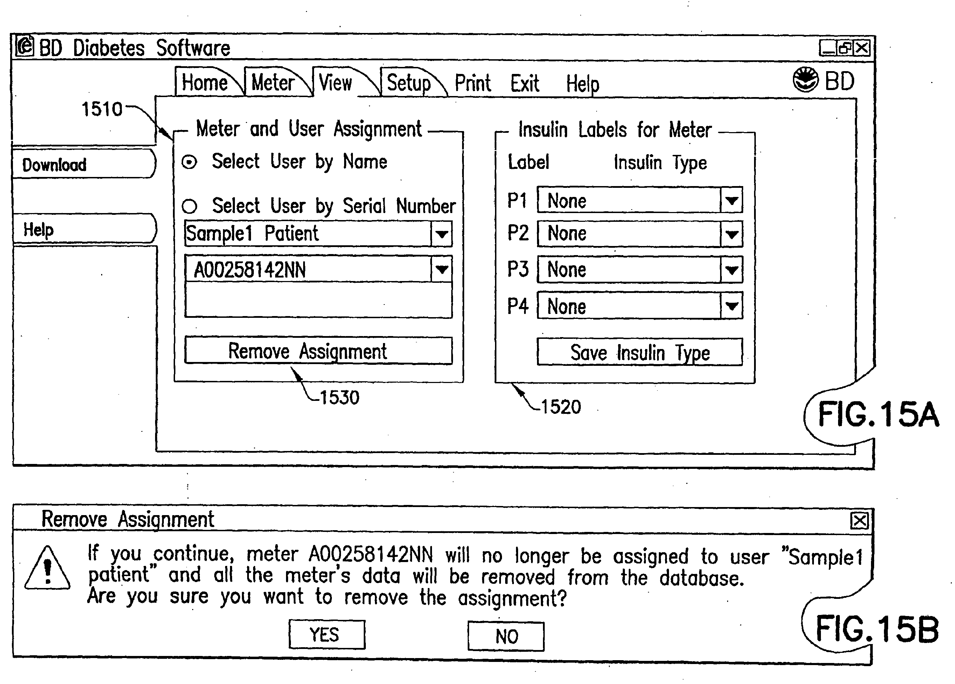

To insure that downloaded measured data is properly attributed to the patient in the database, the serial number of each blood glucose meter 30 is assigned to a particular patient. Additionally, once the measured data is downloaded to the computer the specific measurement is given a tag that preferably includes a unique meter identifier, so every measurement can be attributed to a single meter. When a user downloads data from a blood glucose meter into the software, the meter's unique serial number is transmitted as part of the stream. This serial number is printed on the bottom of the meter. If the software does not recognize the unique serial number as previously downloaded and assigned to a patient, the image shown in Fig. 15 is displayed to the user.

The Meter Page shown in Fig. 15A prompts the user to select a patient user by name or select a meter by serial number 1510. Thereafter, when that meter's data is downloaded, it automatically stores the data in the database, assigned to the previously chosen patient. The person making the assignment is also given the option of entering the insulin labels for the meter 1520. This preferably allows the meter to monitor several types of insulin injected for the particular patient.

The software allows any number of meters to be assigned to a single patient. At least two scenarios exist when a user might want or need to unassign a meter from a patient: (1) at the first download, the user chooses an existing patient by mistake, or (2) the meter is no longer used by one patient and is given to another patient for use. In either scenario, it is necessary to unassign the meter from a particular patient. This is important from a clinical perspective in that clinical decisions could be made for a given patient based on data that does not belong to that patient. Fig. 15B shows the image of the display screen that is preferably presented to the user when the user chooses to remove an assignment.

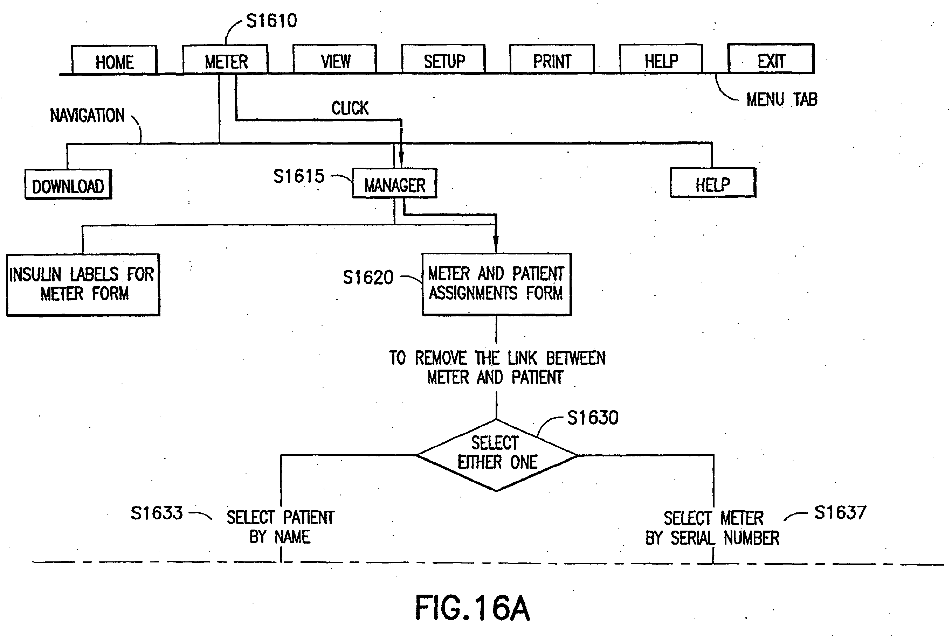

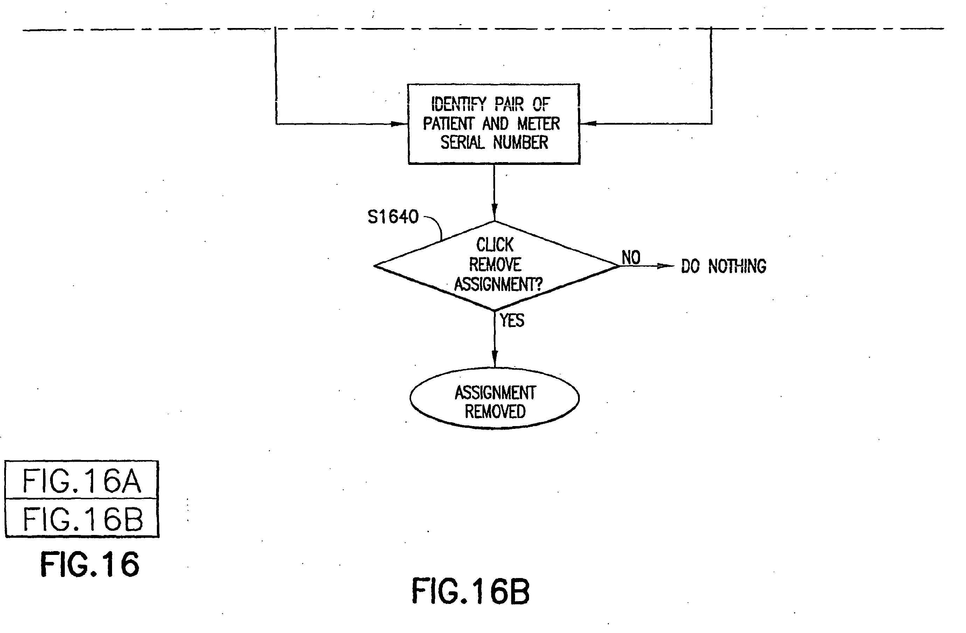

Embodiments of the present invention provide a means for performing this unassignment task. Fig. 16 is a flow chart of the steps required by a user to perform the unassignment tasks. Starting by selecting the Meter tab S1610, the user is presented a navigation menu with the option to click the Manager item S1615. After choosing the Manager option, the user is presented with the view of Fig. 15A, where the user preferably chooses between the Meter and Patient Assignments Form or the Insulin Labels for Meter Form S1620. To remove the link between a patient and a meter, the user must select S1630 either to select a patient by name S1633 or select the meter by serial number S1637. If they choose to select patients by name at S1633, a drop down list is activated with a list of the current patients in the system. When the user selects a name from the list, all meters currently assigned to that patient are shown in the display area. If they choose to select meters by serial number at S1637, the drop down list is activated with a list of the current meter serial numbers in the system. Once the pairing of meter and patient is accomplished, the user has the option S1640 to remove the assignment or do nothing. When the user selects a meter serial number from the list, the patient to whom that meter is assigned is shown in the display area. In both cases, the user can then click on the appropriate item in the display area and click the Remove Assignment button 1530. The user receives a prompt regarding the implication of this choice, an example of which is shown in Fig. 15B and can select either Yes or No. If they select Yes, the assignment is removed and any data received from that meter are no longer available. If they select No, they are returned to the screen and no action is taken.

The components of the software will now be described in more detail. The Meter Manager follows a component oriented architecture, leveraging the Microsoft 'ActiveX' standard and implementing standard software interfaces in order to allow the component to plug into the software's framework/shell. It communicates with database objects via XML transactions. Database updates for meter and patient assignment are accomplished via mapping tables in the database. This makes database updates very quick, and allows assignment and re-assignment of patients across a large dataset to be made quickly, with no decrease in performance as the size of the datasets increase.

A common problem in software applications is the user's inability to find and understand the functions and commands that execute the tasks they wish to accomplish. An embodiment of the present invention to be described next addresses this issue. To solve the problem it is essential to have these kinds of programs available on the first page the user sees upon launching the software and that can be executed by clicking one button. This is accomplished by executing, in a series, the individual processes which: (1) execute a meter download, (2) configure the data displays, and (3) send the configured displays to a printer.

As shown in Fig. 17, the Home Page 1705 feature preferably utilizes HTML to provide the home page functionality. The Home Page 1705 of an embodiment of the present invention preferably has options to click on Meter Download and Printing 1710, Meter Download and Screen Review 1720, Learn How 1730, Help 1740, and Visit BD's Website 1750. However, any commonly used features can be implemented for one-click operation. The HTML document 'home.html' is stored under the software's images directory indicated by a registry key value. The values for the images registry key and contents of 'home.html' are set at installation time. The content of 'home.html' defines several DIV tags. These tags are linked into the software through the BDHome.MainPage component. The tags include "downloadprint". When this tag is clicked it fires 'onclick' events into the BDHome.MainPage component. The BDHome.MainPage component utilizes BDObject operation codes to provide the downloading and printing functionality.

The following describes how a patient can connect the meter and generate a report using the data downloaded from the meter. Fig. 18 shows the steps of clicking Meter Download & Print from the Home Page S1810. The software then automatically starts the download process, which begins by checking whether a data transfer cable is connected to the PC S1820. After connecting S1830, the software begins to identify the meter connected to the cable S1840. After identifying a meter, the software then confirms that the meter and patient are assigned to one another S 1850. Once the device verifications have been confirmed the blood sugar data is downloaded S 1860. After the blood sugar data is downloaded, the insulin data is downloaded S 1870. Once the blood sugar and insulin data is downloaded, the software updates the database and a report is printed for the user S 1880.

The printed reports may be the default reports previously described or may be the user-customized SCP, both of which were described above in more detail.

Oftentimes the healthcare professional must be able to print multiple reports for multiple patients. Referring to Fig. 19A, this batch printing function in the software allows users to choose from a number of options from the My Printouts page 1910 including selecting reports for printing from a list of available reports 1933, selecting Self Care Pages (custom, user-defined reports) from a list of available Self Care Pages 1937, selecting a patient whose data will be printed 1930, selecting a date range for the data that will be printed 1935, or, as shown in Fig. 19B, setting a blood sugar target range for the data that will be printed 1940.

Additionally, the user can access a Help system, select and setup the printer to which the reports will be sent, execute the printing, or quit the batch printing function.

As shown in Fig. 19A, the user can select one or more Self Care Pages 1937 and click the [Add>>>] button 1960 to move the selected pages into the Selected Printouts pane 1920. The user can select one or more Other Reports 1933 and click the [Add>>>] button 1960 to move the selected reports into the Selected Printouts pane 1920. As pages and reports are added to the Selected Printouts pane 1920, the list grows. The user can also select one or more Printouts and click the [<<<Remove] button 1965 to remove them from the Selected Printouts pane 1920.

The user can select from the list of available patients 1930 via a drop-down list. Also, the user can click the date range hyperlink 1935 to access the date range selection tool and a blood sugar target setup (see Fig. 19B, 1940). When the date range is changed or the blood sugar targets are changed, those settings are applied to the printouts for the selected patient.

From the Home Page 1705, the user selects the print tab S2010. A fly out menu then offers a choice of My Printouts, which the user selects S2020. The user then has several options for selecting the patients S2030, dates or target blood sugar measurements S2040, which SCP S2050, and the exact report(s) S2060 to be printed. This provides the user with increased flexibility to customize the reporting features of the software. Additionally, the healthcare provider now has the exact same reports for each patient and reduces the need to have to analyze several different reports for each patient since the software is compatible with different manufactures blood glucose meters. Additionally, the patient does not always have to remember which report to bring into the healthcare provider because the healthcare provider can chose the desired report(s) to print by just downloading the data from the blood glucose meter to the healthcare provider's PC.

The details of the software will now be described in greater detail. The BDView.Container is preferably the entry point for printing activities in Abacus. When the command to print a report is requested, the Navigator sends a message to the BDView.Container to the print job. The BDView.Container holds the information about the particular chart or view to be printed. If it is a chart, the BDView.Container packages up that information into XML and passes it to BDView.CPrintDriver. This class prints the header and footer for a chart, and passes the printer's HDC to the appropriate chart component, which then draws the chart onto the HDC's drawing surface. CprintDriver then prints the actual page.

This control implements the BDObject interface. The implementation of this interface controls the interaction of the control with the navigation framework. The BDView.Container utilizes the following BDObject operation codes to provide system functionality:

| | |
|---|---|
| BDOP_INIT | Intializes new CPatientInfo instance if not batch printing |
| BDOP_STOP | Fires BDOP_STOP, BDOP_TERM, to each loaded control and unloads them. |
| BDOP_TERM | Releases global interfaces |
| BDOP_SETPATIENT | Set the Default Patient Info |
| BDOP_PRESHOW | Setup for display, initializes a report |
| BDOP_POSTSHOW | Position the report client on the screen |
| BDOP_PREHIDE | Hides the header |
| BDOP_POSTHIDE | Close the report |
| BDOP_STARTPRINT | Setup the current control for printing |
| BDOP_SETDATA | Sets a new patient on the current displayed object or batched request object |
| BDOP_PRINT | Setup for printing the current displayed object or the batched |
| | request object |
| BDOP_ENDPRINT | Cleanup after print request |

The BDView.CPrintDriver class controls generation of view reports. The control formats the header and footer information, defines rectangles for chart components and invokes the appropriate chart component to draw output in the specified rectangle. After the chart components draw their output, BDView.CPrintDriver outputs the page(s).

BDView.dlgBatchPrint dialog provides the user interface allowing users to specific a set of reports for batch print requests.

The BDView.dlgBatchPrint component uses the XMLTrans components ("BDViewUtil.XmlTrans") to fetch report information from the BDCustomReport business object. The following transactions are used to Enumerate report data from the BDCustomReport business object:

| | | |
|---|---|---|
| Function | ProgID | BDResource.BDTagID |
| Enumerate | "BD.CustomReportObj" | TRANS_GETREPORTLIST |

Another embodiment of the present invention is preferably embodied in software, and can include, among other things, interactive tutorials specifically designed to instruct users on how to perform two tasks. First, how to connect a blood glucose meter to their computer using either a 9-pin serial cable or a USB cable. Second, how to create a customized Self Care Page for their use.

Referring back to Fig. 17, the tutorials are preferably available from the Home page 1705 of the software. The Home page 1705 contains a "Learn How" button 1730 that when moused over, preferably displays a fly-out menu listing the available tutorials, "Connect the Meter to a PC" and "Create a Self Care Page". The user can click on either of these items to launch a new window in which the selected interactive tutorial executes.

Each tutorial takes the user through the specific steps involved in executing the described function. At any time, the user can click a Next button or a Back button or a Start Again button to navigate the tutorial.

To exit the tutorial, the user preferably clicks the x-button in the upper right of the tutorial window.

Fig. 21 provides a flow diagram of the navigation of the Home Page link to the tutorials. The user begins at the Home Page S2110 and clicks on "Learn How", S 2120. The user must choose between tutorials for connecting the meter to a PC S2131 and creating a self care page S2137. If the user chooses to learn how to connect the meter to a PC, the browser launches a file, preferably a Macromedia Flash file, S2141 for the tutorial that teaches connecting the meter to the PC. Then a self-contained animation is executed S2151 teaching how to connect a meter to a PC. [00101] But if the user selects the create a SCP S2137 from the Home Page S2110, then the browser launches a file, preferably a Flash file, S2141 for the tutorial that teaches how to create a SCP S2147. Then a self-contained animation is executed S2157 explaining the steps for creating a SCP. During the tutorial process, the user may leave the tutorial perform the step just taught and return to the tutorial at the point where the user left. This allows the user to follow the tutorial step by step with out having be concerned about losing his or her place in the tutorial or starting the tutorial over completely.

These interactive tutorials provide simple, step-by-step instructions with animations. The user is able to access these tutorials from the first screen encountered after launching the software. Additionally, the user can continue to use the software while the tutorial is running allowing the user to perform the steps described in the tutorial while the tutorial is running in the background.

The tutorials are preferably executed as Macromedia Flash animations and thus, are launched in a small, controlled, web browser window. The steps involves in with selecting a tutorial will now be described in more detail regarding the software routines that are specifically involved.

As the Home Page is preferably implemented as a basic web page, using HTML, mouse-over and mouse-click actions are defined in the page language for each of the buttons/functions. When the user moves the cursor over the "Learn How" button, the HTML triggers an image change and a fly-out menu display. Each of the items in the fly-out menu can be moused over and an image change is triggered for them as well.

When one of the fly-out menu items is clicked, an event is triggered this launches an instance of the web browser control, with a specific pixel height and width, and now browser controls showing. Inside of this browser instance, a Macromedia Flash file is executed. The Macromedia Flash player is installed when the software is installed, if the installation detects that the user's PC does not have a current version of the player.

With respect to batch printing, the solutions offered in this invention are achieved by combining components of the software that exist in other places within the software with typical batch printing functionality. Furthermore, the user is presented with the same patient selection mechanism and the same date range and blood sugar target mechanism that exists in the rest of the software. This provides additional simplicity in the selection of reports for printing. Ultimately, a user who desires only printed reports, with no on-screen viewing, can use this function to print reports for numerous patients, while having the flexibility of selecting date ranges and blood sugar targets unique to each patient, all from one functional window.

The above described features of the instant invention are exemplary features and are not meant to limit the invention. The above features may be combined in a single software package or made available individually or to only limited users, such as healthcare providers. Additionally, the software may be packaged with hardware products such as a data transfer cable. Further more, the present invention should not be limited to use only with a blood glucose meter as it would be obvious to one of ordinary skill in the art that other devices may require the same functionality of the embodiments of the present invention.

## Claims

1. A method for managing the presentation of medical data comprising:
selecting at least one graphical data chart type from a plurality of graphical display chart types for viewing;
choosing at least one placement location of said at least one selected graphical data chart type from of a plurality of graphical data placement locations in a single visual display;
downloading medical data from a medical device; and
viewing said medical data presented in said selected at least one graphical display chart type in said at least one chosen placement location in said single visual display.

2. The method for managing the presentation of medical data of claim 1, wherein:
the functionality of each graphical data chart type is the same regardless of the placement location of said graphical data chart within the single visual display.

3. The method for managing the presentation of medical data of claim 1, further comprising:
storing the view of said medical data presented in said selected at least one graphical display chart type in said at least one chosen placement location in said single visual display for future use.

4. A method for managing the presentation of medical data comprising:
selecting at least one graphical data chart type from a plurality of graphical display chart types for viewing;
choosing the placement of said at least one selected graphical data chart type from of a plurality of graphical data placement locations in at least one single visual display;
downloading medical data from at least one of a plurality of medical devices; and
printing said downloaded medical data from said at least one of a plurality of medical devices, wherein said downloaded medical data is presented in said at least one graphical display chart in said at least one single visual display.

5. The method for managing the presentation of medical data of claim 4, wherein the printing step further comprises:
storing said medical data presented in said selected at least one graphical display chart type in said at least one chosen placement location in said single visual display into a storage device;
signaling a computer containing said storage device with a single mouse click to receive downloaded data from a medical device and place the new data into the graphical display chart types stored in said storing step; and
printing said at least one single visual display containing the said downloaded data.

6. The method for claim 4, wherein at least one single visual display may be chosen from a group of three default single visual displays or a plurality of user-defmed single visual displays.

7. A method for managing the presentation of medical data comprising the steps of:
selecting at least one graphical data chart type from a plurality of graphical display chart types for viewing;
choosing the placement of said at least one selected graphical data chart type from a plurality of graphical data placement locations in a single visual display;
assigning at least one medical device from a plurality of medical devices to a particular user;
downloading medical data from said at least one of a plurality of medical devices; and
viewing said medical data from said at least one of a plurality of medical devices presented in said at least one graphical display chart type in said single visual display.

8. The method for managing the presentation of medical data of claim 7, further comprising the step of reversing the assignment made in the assigning step.

9. A method for managing the presentation of medical data comprising:
selecting at least one graphical data chart type from a plurality of graphical display chart types representing measured medical data of at least one of a plurality of users;
choosing at least one of a plurality of users for whom a graphical display chart types is configured and saved; and
printing said at least one selected graphical display chart types for each of chosen the plurality of users.

10. The method for managing the presentation of medical data of claim 9 comprising:
selecting a single display showing a plurality of graphical charts representing a plurality of types of medical data;
wherein a first graphical display chart for said single display shows the average, minimum, maximum and standard deviation at predetermined time periods of one of said plurality of types of medical data, wherein said one of said plurality of medical data was measured by a medical device;
wherein a second graphical display chart for said single display shows the minimum dose, maximum dose, average dose and type of medication administered to a user of said medical device; and
wherein further said first and second graphical display charts are presented one above the other in said single display.

11. A method for managing presentation of medical data comprising:
presenting medical data from over a first predetermined time period in a first graphical chart on a display;
indicating on the display a second predetermined time period with a displayed graphical representation; and
creating a second graphical display from the data within the second predetermined time period.

12. The method for managing presentation of medical data of claim 11 further comprising the steps of:
overlaying said second graphical display over said first graphical chart on a display.

13. The method for managing presentation of medical data of claim 11, wherein said second predetermined time period is shorter than said first predetermined time period.

14. The method of managing presentation of medical data of claim 11, wherein said second graphical chart is different from said first graphical chart.

15. The method for managing presentation of medical data of claim 11, wherein said displayed graphical representation is a horizontal bar.

16. A method for managing presentation of medical data comprising:
selecting at least one patient from a plurality of patients in a database;
choosing a print option to print out at least one predetermined graphical chart from a plurality of graphical charts of said medical data corresponding to each patient, wherein said at least one predetermined graphical chart is selected from the group of graphical charts consisting of histogram charts, bar charts, scatter charts, standard deviation charts, standard day charts, and pie charts; and
printing said predetermined graphical charts for said selected patients without any other user input than said choosing step.

17. The method for managing presentation of medical data of claim 16, wherein at least one patient in said selecting step is any number of patients up to all of the patients in said database.

18. The method for managing presentation of medical data of claim 16, wherein said at least one predetermined graphical chart includes all of the graphical charts available.

19. A method for managing presentation of medical data of claim 16, wherein said choosing step further comprises:
actuating a single input to a software program to begin the printing step.

20. An apparatus for presenting medical data comprising:
means for selecting at least one graphical data chart type from a plurality of graphical display chart types for viewing;
means for choosing at least one placement location of said at least one selected graphical data chart type from of a plurality of graphical data placement locations in a single visual display;
means for downloading medical data from a medical device; and
means for viewing said medical data presented in said selected at least one graphical display chart type in said at least one chosen placement location in said single visual display.

21. The apparatus of claim 20, wherein the functionality of each graphical data chart type is the same regardless of the placement location of said graphical data chart within the single visual display.

22. The apparatus of claim 20, further comprising:
means for storing the view of said medical data presented in said selected at least one graphical display chart type in said at least one chosen placement location in said single visual display for future use.

23. An apparatus for presenting medical data comprising:
means for selecting at least one graphical data chart type from a plurality of graphical display chart types for viewing;
means for choosing the placement of said at least one selected graphical data chart type from of a plurality of graphical data placement locations in at least one single visual display;
means for downloading medical data from at least one of a plurality of medical devices; and
means for printing said downloaded medical data from said at least one of a plurality of medical devices, wherein said downloaded medical data is presented in said at least one graphical display chart in said at least one single visual display.

24. The apparatus of claim 23, further comprising:
means for storing said medical data presented in said selected at least one graphical display chart type in said at least one chosen placement location in said single visual display into a storage device;
means for signaling a computer containing said storage device with a single mouse click to receive downloaded data from a medical device and place the new data into the graphical display chart types stored in said storing step; and
means for printing said at least one single visual display containing the said downloaded data.

25. The apparatus of claim 23, further comprising:
means for choosing at least one single visual display from a group of three default single visual displays or a plurality of user-defined single visual displays.

26. An apparatus for presenting medical data comprising:
means for selecting at least one graphical data chart type from a plurality of graphical display chart types for viewing;
means for choosing the placement of said at least one selected graphical data chart type from a plurality of graphical data placement locations in a single visual display;
means for assigning at least one medical device from a plurality of medical devices to a particular user;
means for downloading medical data from said at least one of a plurality of medical devices; and
means for viewing said medical data from said at least one of a plurality of medical devices presented in said at least one graphical display chart type in said single visual display.

27. The apparatus of claim 26, wherein:
said assigning means is adapted to reverse the assignment of at least one medical device from a plurality of medical devices to a particular user.

28. An apparatus for presenting medical data comprising:
means for selecting at least one graphical data chart type from a plurality of graphical display chart types representing measured medical data of at least one of a plurality of users;
means for choosing at least one of a plurality of users for whom a graphical display chart type is configured and saved; and
means for printing said at least one selected graphical display chart types for each of chosen the plurality of users.

29. The apparatus of claim 28, wherein:
said selecting means is adapted to select a single display showing a plurality of graphical charts representing a plurality of types of medical data;
a first graphical display chart for said single display shows the average, minimum, maximum and standard deviation at predetermined time periods of one of said plurality of types of medical data, wherein said one of said plurality of medical data have been measured by a medical. device;
a second graphical display chart for said single display shows the minimum dose, maximum dose, average dose and type of medication administered to a user of said medical device; and
said first and second graphical display charts are presented one above the other in said single display.

30. An apparatus for presenting medical data comprising:
means for presenting medical data from a first predetermined time period in a first graphical chart on a display;
means for indicating on the display a second predetermined time period with a displayed graphical representation;
means for creating a second graphical display from the data within the second predetermined time period.

31. The apparatus of claim 30 further comprising:
means for overlaying said second graphical display over said first graphical chart on a display.

32. The apparatus of claim 30, wherein said second predetermined time period is shorter than said first predetermined time period.

33. The apparatus of claim 30, wherein said second graphical chart is different from said first graphical chart.

34. The apparatus of claim 30, wherein said displayed graphical representation comprises a horizontal bar.

35. An apparatus for presenting medical data comprising:
means for selecting at least one patient from a plurality of patients in a database;
means for choosing a print option to print out at least one predetermined graphical chart from a plurality of graphical charts of said medical data corresponding to each patient, wherein said at least one predetermined graphical chart is selected from the group of graphical charts consisting of histogram charts, bar charts, scatter charts, standard deviation charts, standard day charts and pie charts; and
means for printing said predetermined graphical charts for said selected patients without any other user input than said choosing step.

36. The apparatus of claim 35, wherein at least one patient selected by said selecting means can be any number of patients up to all of the patients in said database.

37. The apparatus of claim 35, wherein said at least one predetermined graphical chart includes all of the graphical charts available.

38. The apparatus of claim 35, wherein said choosing means further comprises:
means for actuating a single input to a software program to begin the printing step.

39. A computer-readable medium having computer-executable instructions arranged for performing a method of managing the presentation of medical data comprising the steps of:
selecting at least one graphical data chart type from a plurality of graphical display chart types for viewing;
choosing at least one placement location of said at least one selected graphical data chart type from of a plurality of graphical data placement locations in a single visual display;
downloading medical data from a medical device; and
viewing said medical data presented in said selected at least one graphical display chart type in said at least one chosen placement location in said single visual display.

40. A computer-readable medium having computer-executable instructions arranged for performing the method of managing the presentation of medical data of claim 39, wherein the functionality of each graphical data chart type is the same regardless of the placement location of said graphical data chart within the single visual display.

41. A computer-readable medium having computer-executable instructions arranged for performing the method of managing the presentation of medical data of claim 39, further comprising the step of:
storing the view of said medical data presented in said selected at least one graphical display chart type in said at least one chosen placement location in said single visual display for future use.

42. A computer-readable medium having computer-executable instructions arranged for performing a method of managing the presentation of medical data comprising the steps of:
selecting at least one graphical data chart type from a plurality of graphical display chart types for viewing;
choosing the placement of said at least one selected graphical data chart type from of a plurality of graphical data placement locations in at least one single visual display; downloading medical data from at least one of a plurality of medical devices; and
printing said downloaded medical data from said at least one of a plurality of medical devices, wherein said downloaded medical data is presented in said at least one graphical display chart in said at least one single visual display.

43. A computer-readable medium having computer-executable instructions arranged for performing the method of managing the presentation of medical data of claim 42, wherein the step of printing further comprises:
storing said medical data presented in said selected at least one graphical display chart type in said at least one chosen placement location in said single visual display into a storage device;
signaling a computer containing said storage device with a single mouse click to receive downloaded data from a medical device and place the new data into the graphical display chart types stored in said storing step; and
printing said at least one single visual display containing the said downloaded data.

44. A computer-readable medium having computer-executable instructions arranged for performing the method of claim 42, wherein at least one single visual display is chosen from a group of three default single visual displays or a plurality of user-defined single visual displays.

45. A computer-readable medium having computer-executable instructions arranged for performing a method of managing the presentation of medical data comprising the steps of:
selecting at least one graphical data chart type from a plurality of graphical display chart types for viewing;
choosing the placement of said at least one selected graphical data chart type from a plurality of graphical data placement locations in a single visual display;
assigning at least one medical device from a plurality of medical devices to a particular user;
downloading medical data from said at least one of a plurality of medical devices; and
viewing said medical data from said at least one of a plurality of medical devices presented in said at least one graphical display chart type in said single visual display.

46. A computer-readable medium having computer-executable instructions arranged for performing the method of managing the presentation of medical data of claim 45, further comprising the step of:
reversing the assignment made in the assigning step.

47. A computer-readable medium having computer-executable instructions arranged for performing a method of managing the presentation of medical data comprising the steps of:
selecting at least one graphical data chart type from a plurality of graphical display chart types representing measured medical data of at least one of a plurality of users;
choosing at least one of a plurality of users for whom a graphical display chart types is configured and saved; and
printing said at least one selected graphical display chart types for each of chosen the plurality of users.

48. A computer-readable medium having computer-executable instructions arranged for performing the method of managing the presentation of medical data of claim 47 comprising the steps of:
selecting a single display showing a plurality of graphical charts representing a plurality of types of medical data;
wherein a first graphical display chart for said single display shows the average, minimum, maximum and standard deviation at predetermined time periods of one of said plurality of types of medical data, wherein said one of said plurality of medical data have been measured by a medical device;
wherein a second graphical display chart for said single display shows the minimum dose, maximum dose, average dose and type of medication administered to a user of said medical device; and
wherein further said first and second graphical display charts are presented one above the other in said single display.

49. A computer-readable medium having computer-executable instructions arranged for performing a method of managing presentation of medical data comprising the steps of:
presenting medical data from over a first predetermined time period in a first graphical chart on a display;
indicating on the display a second predetermined time period with a displayed graphical representation;
creating a second graphical display from the data within the second predetermined time period.

50. A computer-readable medium having computer-executable instructions arranged for performing the method of managing presentation of medical data of claim 49 further comprising the steps of:
overlaying said second graphical display over said first graphical chart on a display.

51. A computer-readable medium having computer-executable instructions arranged for performing the method of managing presentation of medical data of claim 49, wherein said second predetermined time period is shorter than said first predetermined time period.

52. A computer-readable medium having computer-executable instructions arranged for performing the method of managing presentation of medical data of claim 49, wherein said second graphical chart is different from said first graphical chart.

53. A computer-readable medium having computer-executable instructions arranged for performing the method of managing presentation of medical data of claim 49, wherein said displayed graphical representation comprises a horizontal bar.

54. A computer-readable medium having computer-executable instructions arranged for performing a method of managing presentation of medical data comprising the steps of:
selecting at least one patient from a plurality of patients in a database;
choosing a print option to print out at least one predetermined graphical chart from a plurality of graphical charts of said medical data corresponding to each patient, wherein said at least one predetermined graphical chart is selected from the group of graphical charts consisting of histogram charts, bar charts, scatter charts, standard deviation charts, standard day charts and pie charts; and
printing said predetermined graphical charts for said selected patients without any other user input than said choosing step.

55. A computer-readable medium having computer-executable instructions arranged for performing the method of managing presentation of medical data of claim 54, wherein at least one patient in said selecting step is any number of patients up to all of the patients in said database.

56. A computer-readable medium having computer-executable instructions arranged for performing the method of managing presentation of medical data of claim 54, wherein said at least one predetermined graphical chart is the number of graphical charts between one and all of the graphical charts available.

57. A computer-readable medium having computer-executable instructions arranged for performing the method of managing presentation of medical data of claim 54, wherein said choosing step further comprises:
actuating a single input to a software program to begin the printing step.
